# EUROPEAN PATENT APPLICATION

(11) **EP 4 321 869 A1**
(43) Date of publication of application: **14.02.2024**
(21) Application number: 22785053.4
(22) Date of filing: 08.04.2022
(51) Int. Cl.: G01N 33/68, G01N 33/66, C12Q 1/6883, G01N 21/64, A61B 5/1455, A61B 5/00, G01N 30/02

(54) **BIOMARKER FOR NEURODEGENERATIVE DISEASES COMPRISING GLYCOTOXIN, SPECIFIC PROTEIN BOUND TO GLYCOTOXIN, OR GLYCOTOXIN-SPECIFIC PROTEIN COMPLEX**

(30) Priority: 09.04.2021 KR 20210046500; 27.10.2021 KR 20210144355
(71) Applicant: Gachon University of Industry-Academic Cooperation Foundation, Seongnam-si, Gyeonggi-do 13120 (KR); Korea Veterans Health Service, Wonju-si Gangwon-do 26465 (KR)
(72) Inventor: KIM, Sun Yeou, Seoul 06280 (KR); CHANG, Keun-A, Seoul 07987 (KR); SEO, Seung Young, Incheon 22001 (KR); KANG, Min Cheol, Incheon 21932 (KR); KANG, Min Ju, Seongnam-si Gyeonggi-do 13441 (KR); SHIM, Kyu Hwan, Seoul 05721 (KR); JEONG, Da-Eun, Yongin-si Gyeonggi-do 16866 (KR)
(74) Representative: Office Freylinger
(86) International application number: PCT/KR2022/095081
(87) International publication number: WO 2022/216145

(57) **Abstract**

The present invention relates to a biomarker for neurodegenerative diseases, comprising glycotoxin, a specific protein bound to glycotoxin, or a glycotoxin-specific protein complex, and more specifically, the biomarker permits non-invasive rapid detection of a specific fluorescence from the skin surface and therefore can be used in early detection of cognitive impairment, and for a confirmed case of cognitive impairment, measures the mRNA expression level and protein level of the biomarker from living tissues such as skin and blood, and therefore is advantageously utilized in prediction, diagnosis, and the like of neurodegenerative diseases such as Alzheimer's disease and the like.

## Description

### BACKGROUND OF THE INVENTION

### 1. Field of the Invention

The present invention relates to a biomarker for neurodegenerative diseases, comprising glycotoxin, a specific protein bound to glycotoxin, or a glycotoxin-specific protein complex, and more specifically, the biomarker permits non-invasive rapid detection of a specific fluorescence from the skin surface and therefore can be used in early detection of cognitive impairment, and for a confirmed case of cognitive impairment, measures the mRNA expression level and protein level of the biomarker from living tissues such as skin and blood, and therefore is advantageously utilized in prediction, diagnosis, and the like of neurodegenerative diseases such as Alzheimer's disease (AD) and the like.

### 2. Description of the Related Art

Recently, with the development of medicine, the average lifespan of humans is increasing, and new social issues are emerging due to the growing number of elderly people. In particular, senile neurodegenerative diseases such as Alzheimer's disease (AD) and Parkinson's disease appear as fatal functional disorders of the nervous system, and there is no effective method for treating them until now.

In particular, one of the most commonly observed senile neurodegenerative diseases is Alzheimer's disease (AD). Alzheimer's disease (AD) is the most common degenerative brain disease causing dementia. It leads to problems in memory, thinking, and behavior, resulting in a significant loss of memory and other cognitive abilities to the extent that it interferes with daily life.

The exact cause and pathogenesis of Alzheimer's disease (AD) are not well understood, but damage to neurons due to decreased synthesis of acetylcholine, a neurotransmitter, deposition of β-amyloid, and hyperphosphorylation of tau protein is thought to be the main cause.

Early diagnosis of these neurodegenerative diseases is becoming increasingly important, as early detection and treatment can reduce societal costs. However, until now, the clinical diagnosis of Alzheimer's disease has relied primarily on medical history and neuropsychological examination, with imaging tests such as magnetic resonance imaging (MRI) and positron emission tomography (PET) as secondary tests. MRI and PET are methods used to diagnose Alzheimer's disease (AD) by assessing the accumulation level of amyloid beta in the brain through brain imaging, but they have limitations such as high costs and difficulty in universal application, making it difficult for most patients to undergo the tests. Accordingly, there is a need for a method for diagnosing neurodegenerative diseases such as Alzheimer's disease (AD) quickly and conveniently and non-invasively.

In the human body, advanced glycation end products (AGEs) are formed through the non-enzymatic reaction of sugar and protein side chains. This process begins with a reversible reaction between a reducing sugar and an amino group that forms a Schiff base, and continues to form a covalently-bonded Amadori rearrangement product. Once the Amadori product is formed, it undergoes further rearrangement, and generates advanced glycation end-products (AGEs).

AGEs can also be formed from other processes. For example, the advanced glycation end-product, Nε-(carboxylmethyl) lysine, is a product of both lipid peroxidation and glycoxidation reactions.

Until now, dozens of advanced glycation end-products are known, and examples thereof include pentosidine, carboxymethyl lysine (CML), carboxyethyl lysine (CEL), pyrraline, OMA (oxalic acid monolysinylamide), imidazolones, GLAP (glyceraldehydes-derived pyridinum compound), GOLD (glyoxal-lysine dimer), MOLD (methyl-glyoxal-lysine dimmer), crossline, and FFI(2-(2-furoyl)-4(5)-(2-furanyl)-1H-imidazole).

In general, advanced glycation end-products (AGEs) accumulate in the body at a rate proportional to the average level of blood sugar. Advanced glycation end-products (AGEs) are produced and accumulated by normal metabolism and aging, and the accumulation of advanced glycation end-products (AGEs) increases in proportion to age and remains in the body for a long time. In particular, when hyperglycemia caused by diabetes persists, a large amount of advanced glycation end-products (AGEs) accumulates in the body. The accumulation of these advanced glycation end-products in the body promotes post-translational modification of membrane proteins.

When the hyperglycemic state persists, the reversible Amadori-type early glycation products are not degraded but rearranged and cross-linked with long-lived proteins such as collagen to form irreversible advanced glycation end-products (AGEs).

In addition, the advanced glycation end-products produced in this way form irreversible cross-links with long-lived matrix proteins such as collagen, laminin, and fibronectin, as well as cross-links with extracellular matrix proteins, reactions with cellular advanced glycation end-product receptors, and advanced glycation end-products with intracellular proteins or nucleic acids, leading to diabetic complications. One reason for this is that normal cross-linking of collagen usually occurs only at specific sites at the N- and C-termini, while cross-linking of collagen by advanced glycation end-products occurs randomly at all sites.

These irreversible, long-lived advanced glycation end-products play a critical role in several diseases. For example, many diabetic patients are unable to prevent complications and end up suffering from blindness, nephropathy, stroke, and amputation, which shows that preventing or treating complications is not just about controlling blood sugar level, but also about reducing the irreversible and long-lived advanced glycation end-products that have already accumulated.

In addition, the formation of advanced glycation end-products is accompanied by an increase in free radical activity and changes in the structure and function of proteins, which may contribute to the development of neurodegenerative diseases. In Alzheimer's disease (AD), advanced glycation end-products (AGEs) can be detected in pathological deposits such as amyloid plaques and neurofibrillary tangles and can explain many of the neuropathological and biochemical characteristics of Alzheimer's disease (AD) .

In addition, AGEs are associated with several pathological conditions, including inflammation, retinopathy, nephropathy, atherosclerosis, stroke, endothelial cell dysfunction, and neurodegenerative disease.

Collagen is the most common protein in the skin and is easily glycated, and it changes with age along with the amount of advanced glycation end-products (AGEs) that accumulate in the skin. Glucose binds to proteins through a non-enzymatic process known as glycation to form covalent adducts, and some of these protein cross-linked sites fluoresce.

Skin collagen advanced glycation end-products often take the form of fluorescent cross-links and adducts, and the accumulated advanced glycation end-products in skin tissues can be relatively easily and noninvasively assessed by measuring skin autofluorescence (SAF).

Recently, it has been reported that the accumulation of carboxymethyl lysine (CML) and pentosidine in the skin is positively correlated in diabetic patients, and that the development of diabetic complications can be predicted by measuring advanced glycation end-products in diabetic patients by identifying the increased accumulation of advanced glycation end-products due to diabetes (Meerwaldt R, Graaff R, Oomen PH, et al. Simple non-invasive assessment of advanced glycation end-product accumulation. Diabetologia. 2004;47:1324-1330).

However, it has not been reported so far whether it is possible to diagnose the onset of neurodegenerative disease by measuring advanced glycation end-products.

Accordingly, the present inventors have completed the present invention by confirming that diseases such as Alzheimer's disease (AD) can be diagnosed by noninvasively measuring the specific fluorescence of glycotoxin, specific protein bound to glycotoxin, or glycotoxin-specific protein complex present in the skin, which are advanced glycation end-products (AGEs), or by measuring the amount of mRNA expression or protein in biological tissues such as skin, fingernails, toenails, hair, blood, and cerebrospinal fluid.

### SUMMARY OF THE INVENTION

It is an object of the present invention to provide a biomarker comprising glycotoxin, a specific protein bound to glycotoxin, or a glycotoxin-specific protein complex, and a method for diagnosing neurodegenerative diseases using the same.

To achieve the above object, the present invention provides a biomarker for diagnosing neurodegenerative diseases, comprising glycotoxin, a specific protein bound to glycotoxin, or a glycotoxin-specific protein complex.

The present invention also provides a method for diagnosing neurodegenerative diseases using a biomarker comprising glycotoxin, a specific protein bound to glycotoxin, or a glycotoxin-specific protein complex.

In addition, the present invention provides a kit for diagnosing neurodegenerative diseases using a biomarker comprising glycotoxin, a specific protein bound to glycotoxin, or a glycotoxin-specific protein complex.

### ADVANTAGEOUS EFFECT

The present invention enables easy diagnosis of neurodegenerative diseases such as Alzheimer's disease (AD) by noninvasively measuring the specific fluorescence of glycotoxin, specific protein bound to glycotoxin, or glycotoxin-specific protein complex present in the skin, or by measuring the amount of mRNA expression or protein in skin tissues and blood, thereby determining the appropriate treatment direction for the patient.

### BRIEF DESCRIPTION OF THE DRAWINGS

Figure 1 is a set of graphs showing the amounts of (a) pentosidine, (b) carboxyethyl lysine (CEL), and (c) carboxymethyl lysine (CML) among the advanced glycation end-products in the skin tissues of the normal group (WT) and the disease group (5xFAD) of Alzheimer's disease model mice, confirmed by HPLC.
Figure 2 is a set of graphs showing the amounts of (a) pentosidine, (b) carboxyethyl lysine (CEL), and (c) carboxymethyl lysine (CML) among the advanced glycation end-products in the plasma of the normal group (WT) and the disease group (5xFAD) of Alzheimer's disease model mice, confirmed by ELISA.
Figure 3 is a set of graphs showing the levels of (a) collagen 1 mRNA expression, (b) collagen 2 mRNA expression, (c) collagen 3 mRNA expression, (d) collagen 4 mRNA expression, (e) collagen 5 mRNA expression, and (f) collagen 6 mRNA expression in the skin tissues of the normal group (WT) and the disease group (5xFAD) of Alzheimer's disease model mice.
Figure 4 is a graph confirming the level of collagen 7 mRNA expression in the skin tissues of the normal group (WT) and the disease group (5xFAD) of Alzheimer's disease model mice.
Figures 5a and 5b are diagrams confirming the level of collagen XVII expression in the skin tissues of the normal group (WT) and the disease group (5xFAD) of Alzheimer's disease model mice.
Figure 6 is a graph confirming the level of collagen XVII mRNA expression in the skin tissues of the normal group (young) and the aged group (old) in aging model mice.
Figure 7 is a set of graphs showing the amounts of (a) pentosidine, (b) carboxyethyl lysine (CEL), (c) carboxymethyl lysine (CML), and (d) MG-H1 in the plasma of the normal group (HC) and the cognitive decline group (CI).
Figure 8 is a graph showing the amount of collagen XVII in the plasma of the normal group (HC) and the cognitive decline group (CI).
Figure 9 is a set of graph showing (a) the amounts of methylglyoxal (MGO) and (b) the enzyme activity glyoxalase-1 (GLO- 1) in the plasma of the normal group (HC) and the cognitive decline group (CI) .
Figure 10 is a set of graphs showing the correlation between the Novel Object Recognition Test (NORT) results for Alzheimer's disease model mice (5xFAD) and the contents of (a) pentosidine, (b) carboxyethyl lysine (CEL), and (c) carboxymethyl lysine (CML) in the skin tissues.
Figure 11 is a graph showing the correlation between the Novel Object Recognition Test (NORT) results for Alzheimer's disease model mice (5xFAD) and the level of collagen XVII mRNA expression.
Figure 12 is a set of graphs showing the correlation between the level of collagen XVII mRNA expression and the contents of (a) pentosidine, (b) carboxyethyl lysine (CEL), and (c) carboxymethyl lysine (CML) in the skin tissues.
Figure 13 is a graph showing the skin autofluorescence (SAF) of the normal group (HC), the diabetic patient group (HCD), the cognitive decline group (CI), and the diabetic • cognitive decline group (CID).
Figure 14 is a graph showing the diagnostic performance through skin autofluorescence between the normal groups (HC + HCD) and the cognitive decline groups (CI + CID).
Figure 15 is a set of graphs showing the correlation between skin autofluorescence (SAF) and clinical indicators in the non-diabetic group, and Figure 15a shows MMSE, Figure 15b shows creatinine, and Figure 15c shows CKD-EPI.
Figure 16 is a set of graphs showing the correlation between skin autofluorescence (SAF) and clinical indicators in the diabetic group, and Figure 16a shows MMSE, Figure 16b shows creatinine, Figure 16c shows CKD-EPI, Figure 16d shows HbA1c, and Figure 16e shows HDL.
Figure 17 is a diagram illustrating the visualized brain region showing the correlation between skin autofluorescence (SAF) and FDG-PET.
Figure 18 is a set of graphs showing the amount of advanced glycation end-products combined with collagen XVII in the plasma of normal group (HC) and cognitive decline group (CI), wherein Figure 18a shows pentosidine, Figure 18b shows carboxyethyl lysine (CEL) and, Figure 18c shows carboxymethyl lysine (CML).
Figure 19 is a set of graphs showing the correlation between the amount of advanced glycation end-products bound to collagen XVII in the plasma of the normal group (HC) and the cognitive decline group (CI) and the Mini-Mental State Examination (MMSE) results, wherein Figure 18a shows pentosidine, Figure 18b shows carboxyethyl lysine (CEL), and Figure 18c shows carboxymethyl lysine (CML).
Figure 20 is a diagram showing the expression patterns of collagen XVII, CEL, and CML in the plasma of the cognitive decline group (CI).
Figure 21 is a graph showing the changes in the ratio of carboxyethyl lysine (CEL) to methylglyoxal hydroimidazolone (MG-H1) in the blood of the normal group (HC), the diabetic patient group (HCD), the cognitive decline group (CI) and the diabetic cognitive decline group (CID).
Figure 21b is a graph showing the changes in the ratio of carboxyethyl lysine (CEL) to methylglyoxal hydroimidazolone (MG-H1) in the blood of the normal group (HC), the diabetic patient group (HCD), the cognitive decline group (CI) and the diabetic cognitive decline group (CID), and clinical dementia rating (CDR).
Figure 21c is a graph showing the correlation between the changes in the ratio of carboxyethyl lysine (CEL) to methylglyoxal hydroimidazolone (MG-H1) in the blood of the normal group (HC), the diabetic patient group (HCD), the cognitive decline group (CI) and the diabetic cognitive decline group (CID), and the Mini-Mental State Examination (MMSE) results.
Figure 22 is a set of graphs showing the performance of (a) CEL, (b) MG-H1, (c) CML, (d) pentosidine, (e) CEL/MG-H1, (f) CML/MG-H1, and (g) pentosidine/MG-H1 as biomarkers in the normal group (HC) and the cognitive decline group (CI), analyzed by ROC curve analysis.
Figure 23 is a set of graphs showing the performance of (a) collagen XVII, (b) collagen XVII-CEL, (c) collagen XVII and collagen XVII-CEL, (d) collagen XVII/MG-H1, (e) collagen XVII_CEL/MG-H1, and (f) collagen XVII/MG-H1 and collagen XVII_CEL/MG-H1 in the blood of the normal group (HC) and the cognitive decline group (CI), analyzed by ROC curve analysis.
Figure 24 is a diagram showing the chemical formulas of glycotoxins (pentosidine, CEL, CML, and MG-H1), glycotoxin-protein complexes (protein-pentosidine, protein-CEL, protein-CML, and protein-MG-H1), and collagen XVII-CEL, along with the binding states of the proteins to the formulas.

### DESCRIPTION OF THE PREFERRED EMBODIMENTS

The present invention provides a biomarker for diagnosing neurodegenerative diseases, comprising glycotoxin, a specific protein bound to glycotoxin, or a glycotoxin-specific protein complex.

The glycotoxin is at least one selected from the group consisting of pentosidine, carboxymethyl lysine (CML), carboxyethyl lysine (CEL), pyrraline, OMA (oxalic acid monolysinylamide), imidazolones, GLAP (glyceraldehydes-derived pyridinum compound), GOLD (glyoxal-lysine dimer), MOLD (methyl-glyoxal-lysine dimmer), crossline, and FFI (2-(2-furoyl)-4(5)-(2-furanyl)-1H-imidazole).

An example of the imidazolone is methylglyoxal hydroimidazolone (MG-H1).

In the present invention, glycotoxin and advanced glycation end-product have the same meaning.

In the present invention, the measured CEL is the Total CEL value, which is the result of including both free CEL and protein-bound CEL.

The specific protein is at least one selected from the group consisting of collagen, elastin, keratin, amyloid β species, tau, alpha-synuclein, and TDP-43.

The collagen is collagen XVIIA.

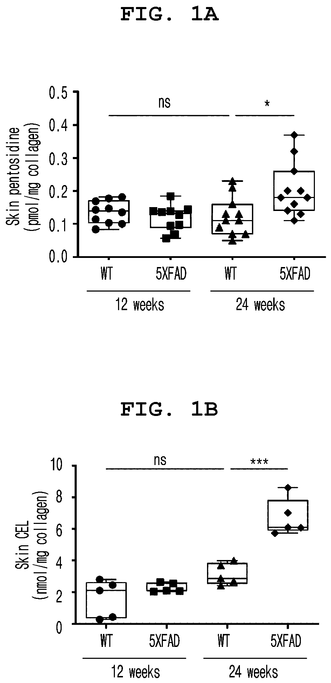

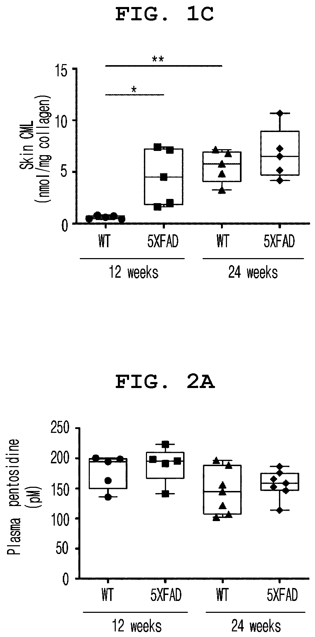

In one experimental example of the present invention, advanced glycation end-products (AGEs) were measured in the plasma and skin tissues of Alzheimer's disease model (5xFAD) mice. As a result, it was confirmed that the amounts of pentosidine, carboxyethyl lysine (CEL), and carboxymethyl lysine (CML) were increased significantly in the skin tissues of the 24-week disease group (5xFAD) (Figures 1a to 1c).

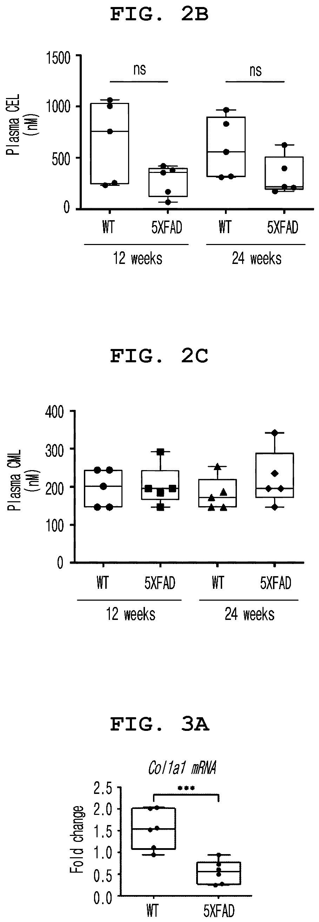

On the other hand, there was no significant difference in the amounts of pentosidine and carboxymethyl lysine (CML) in the plasma of the normal group (WT) and the disease group (5xFAD), and the amount of carboxyethyl lysine (CEL) tended to decrease in both the 12- and 24-week disease groups (5xFAD), but there was no difference between the two groups (Figures 2a to 2c).

This showed that in Alzheimer's disease (AD), one of the representative neurodegenerative diseases, the advanced glycation end-products (AGEs) accumulated in the skin, and they can be used as biomarkers for diagnosis and treatment of Alzheimer's disease (AD).

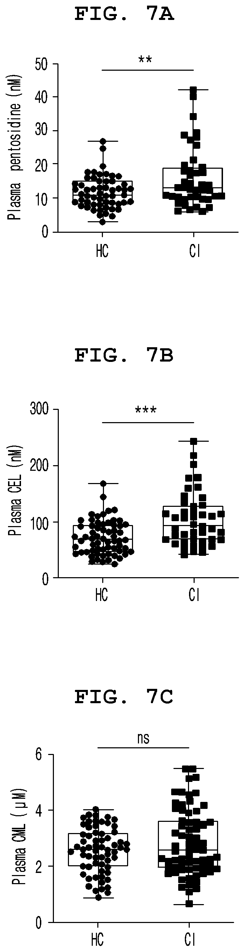

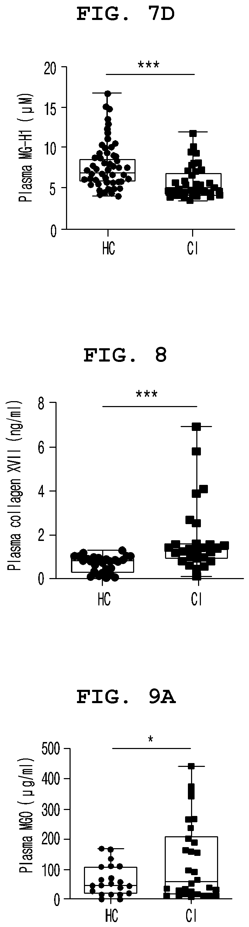

In addition, in one experimental example of the present invention, advanced glycation end-products (AGEs) in the plasma of the normal group (HC) and the cognitive decline group (CI) were measured. As a result, the contents of pentosidine and carboxyethyl lysine (CEL) in the plasma of the cognitive decline group (CI) were increased compared to the normal group (HC) (Figures 7a to 7d). Unlike these increases, however, CML did not increase significantly in the CI group.

This showed that the contents of pentosidine and (carboxyethyl) lysine (CEL), the glycotoxins, were increased in the plasma of patients with cognitive decline. Therefore, they can be targeted and used for diagnosis and treatment of the disease.

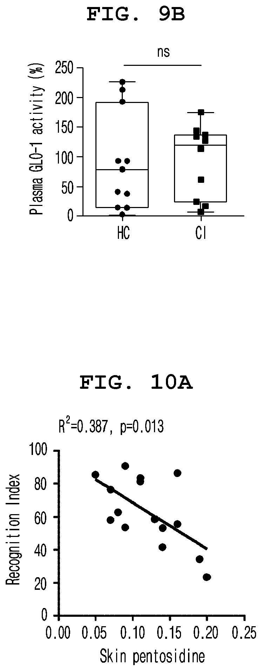

Meanwhile, in one experimental example of the present invention, the enzyme activity of glyoxalase 1 in the blood of the normal group (HC) and the cognitive decline group (CI) was measured. As a result, the enzyme activity of glyoxalase 1 in the plasma of the cognitive decline group (CI) was lower than that in the plasma of the normal group (HC) (Figure 9).

The lower activity of glyoxalase 1, a methylglyoxal lyase, in patients with cognitive decline indicates that methylglyoxal, a precursor of carboxyethyl lysine (CEL), is degraded less, which in turn indicates that the less degraded methylglyoxal in patients with cognitive decline can be converted to carboxyethyl lysine (CEL) more, and consequently, the amount of carboxyethyl lysine can be increased in patients with cognitive decline. These results are consistent with the increase of carboxyethyl lysine (CEL) in the plasma of the cognitive decline group (CI) .

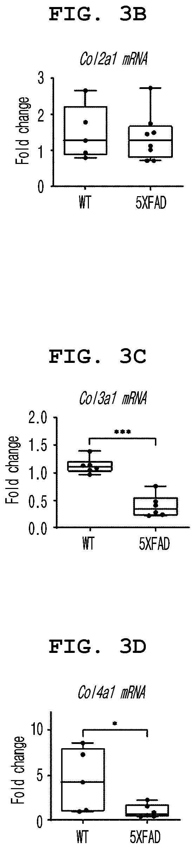

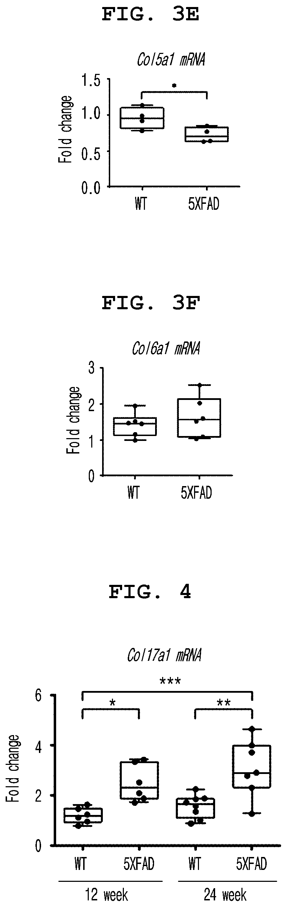

In one experimental example of the present invention, the mRNA expression levels of collagen 1 to 6 and 17 in the skin tissues of Alzheimer's disease model (5xFAD) mice were measured. As a result, the mRNA expression levels of collagen 1, 3, 4, and 5 were decreased in the skin of the disease group (5xFAD), while the mRNA expression levels of collagen 2 and 6 did not show a significant increase (Figures 3a to 3f). On the other hand, it was confirmed that the mRNA expression of collagen XVII in the skin of the disease group (5xFAD) was increased significantly (Figure 4).

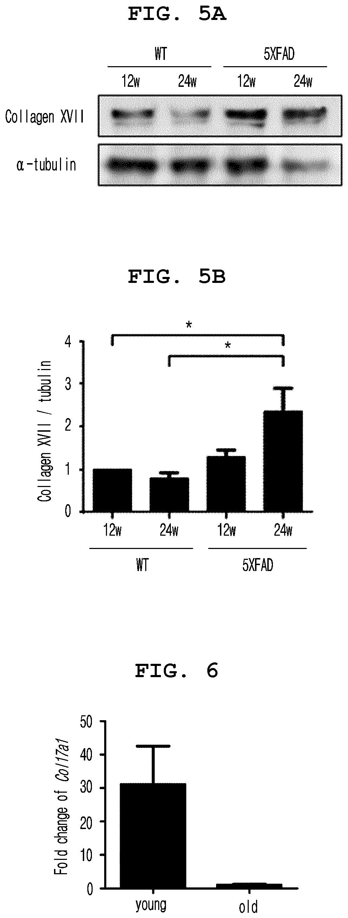

In one experimental example of the present invention, the protein expression level of collagen XVII in the skin tissues of Alzheimer's disease model (5xFAD) mice was measured. As a result, it was confirmed that the protein expression of collagen XVII in the skin of the disease group (5xFAD) was significantly increased (Figure 5).

In addition, in one experimental example of the present invention, the content of collagen XVII in the plasma of the normal group (HC) and the cognitive decline group (CI) was measured. As a result, it was confirmed that the content of collagen XVII in the blood of the cognitive decline group (CI) was significantly increased compared to the normal group (HC) (Figure 8).

This showed that the amount of collagen XVII in the blood was increased in the blood of patients with cognitive decline. Therefore, it can be targeted and used for diagnosis and treatment of cognitive decline.

In addition, in one experimental example of the present invention, the mRNA expression level of collagen XVII in the skin of the aging mouse model (old) was measured. As a result, the mRNA expression level of collagen XVII in the skin was decreased in the aged group (old) compared to the control group (young) (Figure 6).

This indicates that collagen XVII is not simply increased by aging, but is increased in the presence of Alzheimer's disease (AD).

Therefore, it was confirmed that collagen XVII can be used as a biomarker to diagnose and treat Alzheimer's disease (AD), separately from those caused by aging.

The present invention also provides a method for diagnosing neurodegenerative diseases by measuring glycotoxin, a specific protein bound to glycotoxin, or a glycotoxin-specific protein complex in a biological tissue or biological fluid.

The glycotoxin, specific protein bound to glycotoxin, or glycotoxin-specific protein complex is measured by any one or more methods of measuring fluorescence, gene expression level, and protein level.

The glycotoxin can be selected from the group consisting of pentosidine, carboxyethyl lysine (CEL), carboxymethyl lysine (CML) and methylglyoxal hydroimidazolone (MG-H1). The specific protein bound to glycotoxin can be selected from the group consisting of collagen, elastin, keratin, amyloid β species, tau, alpha-synuclein and TDP-43, and is preferably collagen.

The measurement of glycotoxin, a specific protein bound to glycotoxin, or a glycotoxin-specific protein complex is the measurement of a complex of pentosidine, carboxyethyl lysine (CEL) or carboxymethyl lysine (CML) and collagen XVII.

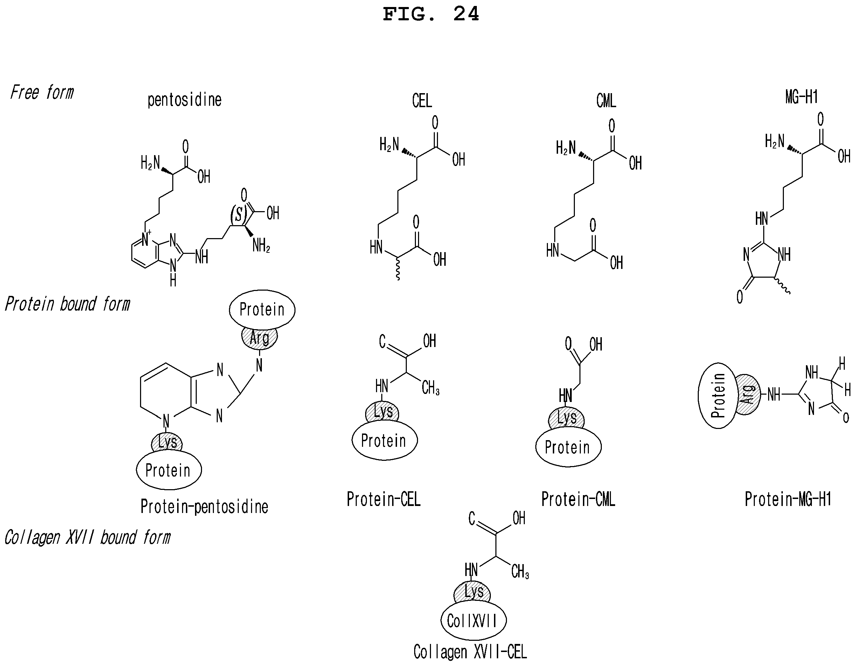

The chemical formulas of glycotoxins (pentosidine, CEL, CML, and MG-H1), glycotoxin-protein complexes (protein-pentosidine, protein-CEL, protein-CML, and protein-MG-H1), and collagen XVII-CEL, along with the binding states of the proteins to the formulas are shown in Figure 24.

The present invention also provides a method for predicting or diagnosing neurodegenerative diseases by measuring the accumulation ratio of glycotoxin, a specific protein bound to glycotoxin, or a glycotoxin-specific protein complex, and methylglyoxal hydroimidazolone (MG-H1).

The accumulation ratio is at least one selected from the group consisting of CEL/MGH1, CML/MGH1, pentosidine/MGH1. collagen XVII/MGH1, collagen XVII-CEL/ MGH1, collagen XVII-CML/MGH1, and collagen XVII-pentosidine/MGH1.

The measurement of the accumulation ratio is a measurement of [any one of CEL/MGH1, CML/MGH1, pentosidine/MGH1, or collagen XVII/MGH1] and [a complex of collagen XVII with at least one selected from the group consisting of pentosidine, carboxyethyl lysine (CEL), and carboxymethyl lysine (CML)].

The biomarker is either CEL/MGH1 or collagen XVII-CEL. In the present invention, the composite biomarker is for the analysis of CEL/MGH1, a ratio of glycotoxin accumulation, and the single biomarker is for the analysis of a collagen XVII-CEL complex.

The neurodegenerative disease is at least one selected from the group consisting of Alzheimer's disease, Parkinson's disease, amyotrophic lateral sclerosis, Huntington's chorea, spinocerebellar degeneration, prion diseases, Creutzfeldt-Jakob disease, frontotemporal dementia, vascular dementia, Lewy body dementia, and dementia with Parkinson's disease.

In one embodiment of the present invention, the correlation between the cognitive decline evaluation results through the Novel Object Recognition Test (NORT) for the Alzheimer's disease model (5xFAD) mice and the biomarkers of the present invention was compared.

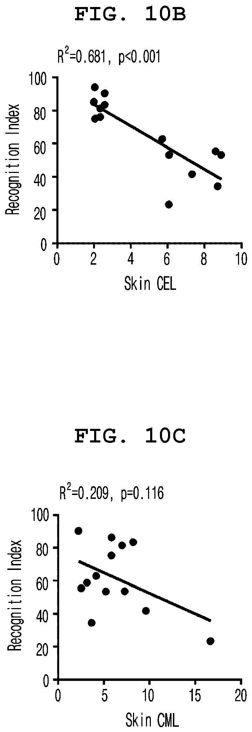

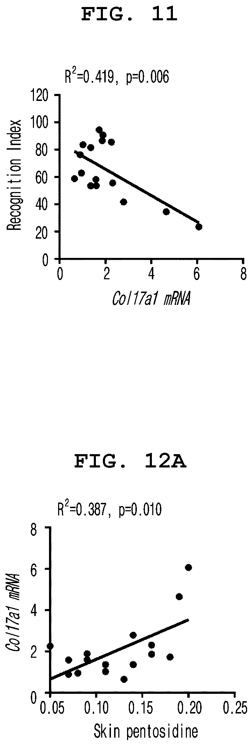

As a result, the content of pentosidine, CEL, and CML in the skin tended to increase as the cognitive ability of the disease model mice decreased, and among them, pentosidine and CEL were highly correlated with cognitive ability (Figures 10a to 10c). In addition, as the cognitive function decreased, the mRNA expression level of collagen XVII in the skin tended to increase (Figure 11).

Through this, it was confirmed that there is a correlation between cognitive decline and pentosidine, carboxyethyl lysine (CEL), and collagen XVII.

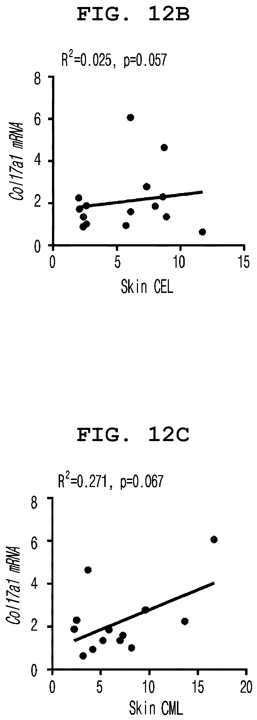

In one experimental example of the present invention, the amount of the advanced glycation end-products bound to collagen XVII in the plasma of the normal group (HC) and the cognitive decline group (CI) was measured. As a result, it was confirmed that CEL bound to collagen XVII in the blood of the cognitive decline group (CI) was significantly increased compared to the normal group (HC) (Figures 12a to 12c) .

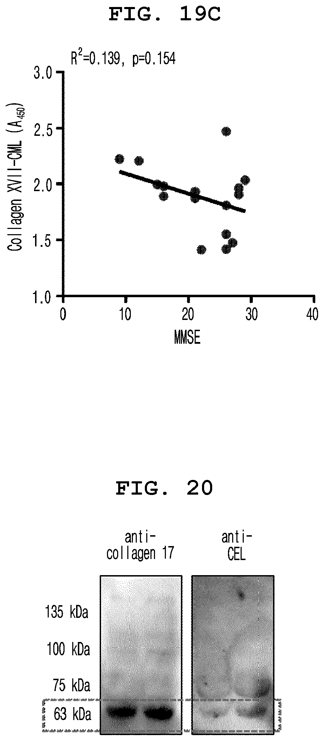

In addition, in one experimental example of the present invention, the expression patterns of collagen XVII and CEL in the plasma of the cognitive decline group (CI) were confirmed. As a result, collagen XVII and CEL were detected at the same protein size location (~70 kDa) among several protein size locations. This indicates that when CEL is present in the blood, it binds to collagen XVII and forms advanced glycation end-products (Figure 20).

This shows that the increased collagen XVII in the blood is selectively bound to CEL rather than other advanced glycation end-products in the presence of cognitive decline such as dementia. Therefore, it can be targeted and used for prediction and diagnosis of the disease.

Measuring glycotoxin, a specific protein bound to glycotoxin, or a glycotoxin-specific protein complex in the biological tissue is to measure skin autofluorescence (SAF).

The skin autofluorescence (SAF) can distinguish the normal group and the cognitive decline group (CI).

In one experimental example of the present invention, the skin autofluorescence (SAF) specific to the biomarker of the present invention was measured in the normal group (HC), the diabetic group (HCD), the cognitive decline group (CI), and the diabetic cognitive decline group (CID) using a noninvasive method.

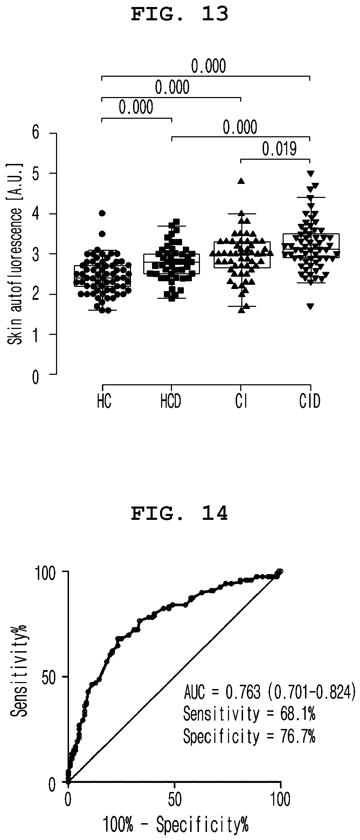

As a result, the skin autofluorescence (SAF) was increased in the diabetic cognitive decline group (CID), the cognitive decline group (CI), and, the diabetic group (HCD) compared to the normal group (HC), and was further increased in the diabetic cognitive decline group (CID) compared to the cognitive decline group (CI) and the diabetic group (HCD), respectively (Figure 13).

The increase in the measured skin autofluorescence (SAF) value can be a major indicator of diabetes, and in the present invention, it was confirmed that the measured value of the skin autofluorescence (SAF) increased even in the cognitive decline group (CI) without diabetes. This means that the measured skin autofluorescence (SAF) value is associated with cognitive decline independently of diabetes. In particular, in the group with both diabetes and cognitive decline (CID), it can be interpreted that the skin autofluorescence (SAF) value is the highest due to the interaction between diabetes and cognitive decline.

Seeing that the SAF value increased to a high level even in the cognitive decline group (CI) without hyperglycemia, it was shown that the increase in glycotoxin in the skin could be a breakthrough marker for neurodegenerative diseases including Alzheimer's disease. In addition, since the highest SAF value was shown in patients with cognitive decline accompanied by diabetes, it showed the possibility of diagnosing neurodegenerative diseases through glycotoxin in the skin of patients with diabetes.

Furthermore, data showing a high correlation with MMSE, creatinine, and CKD-EPI, and no correlation with HbA1c, confirmed that the results of skin autofluorescence (SAF) in non-diabetic subjects (HC and CI) were independent of diabetes, as it was increased specifically in the cognitive decline group (Table 3). In other words, the increase of SAF measured in the skin is a biomarker that independently affects cognitive decline such as Alzheimer's patients, and it is judged to be further accelerated in the presence of diabetes. Therefore, this SAF may be an independent diagnostic marker that can be used to predict early cognitive decline different from diabetes.

In one experimental example of the present invention, variables affecting cognitive decline were analyzed. As a result, in the univariate analysis, it was confirmed that skin autofluorescence, diabetes, and glucose each had an effect on cognitive decline. Among them, skin autofluorescence had the highest odds ratio (Table 4). These results confirm that the degree of skin autofluorescence is a key factor in determining cognitive decline.

In one experimental example of the present invention, the correlation between skin autofluorescence and analytical values quantifying MRI (magnetic resonance imaging) results was analyzed. All indicates the analysis results including all groups (HC, HCD, CI, and CID). According to DM (diabetes mellitus), the analysis was performed by dividing into non-DM (HC+CI) and DM (HCD+CID).

As a result, when analyzed in all groups, significant statistical values were shown in all results except for periventricular white matter hyperintensities (PVWMH) (Table 5).

These results show that skin autofluorescence is sufficiently reflective of substantial changes in the brain. In all experimental groups, skin autofluorescence showed a high correlation with brain atrophy and white matter hyperintensity. In addition, skin autofluorescence reflected brain changes differently depending on the presence or absence of diabetes.

In one experimental example of the present invention, the correlation between FDG(18F-fludeoxyglucose)-PET(positron emission tomography) showing brain glucose metabolism and skin autofluorescence in the Non-DM(HC+CI) group was visualized.

As a result, the correlation between the decreased brain glucose metabolism and the increased skin autofluorescence was strongest in the parietal cortex and the posterior cingulate gyrus.

The metabolic reduction in the parietal cortex coincided with the atrophy results on MRI (magnetic resonance imaging), confirming once again that skin autofluorescence indicates changes in the brain. In particular, the posterior cingulate gyrus is known to be the most important region for diagnosing Alzheimer's disease in FDG-PET. Thus, the correlation between skin autofluorescence and decreased glucose metabolism in the posterior cingulate gyrus suggests that skin autofluorescence reflects the changes in the brain caused by Alzheimer's disease.

The biological tissue is at least one selected from the group consisting of skin, fingernails, toenails, and hair.

The biological fluid is tears, saliva, urine, blood, or cerebrospinal fluid.

The measurement of glycotoxin, a specific protein bound to glycotoxin, or a glycotoxin-specific protein complex in the biological fluid is to measure the gene expression levels and protein levels in the blood.

The present invention also provides a kit for diagnosing neurodegenerative diseases that measures glycotoxin, a specific protein bound to glycotoxin, or a glycotoxin-specific protein complex in the biological tissue or biological fluid.

The glycotoxin that can be used in the kit is at least one selected from the group consisting of pentosidine, carboxymethyl lysine (CML), carboxyethyl lysine (CEL), pyrraline, OMA (oxalic acid monolysinylamide), imidazolones, GLAP (glyceraldehydes-derived pyridinum compound), GOLD (glyoxal-lysine dimer), MOLD (methyl-glyoxal-lysine dimmer), crossline, and FFI (2-(2-furoyl)-4(5)-(2-furanyl)-1H-imidazole).

Imidazolone includes methylglyoxal hydroimidazolone (MG-H1).

The specific protein bound to glycotoxin is at least one selected from the group consisting of collagen, elastin, keratin, amyloid β species, tau, alpha-synuclein, and TDP-4. Collagen includes collagen 17A.

Glycotoxins or proteins bound to glycotoxin may be used alone or in combination.

Specifically, the kit can predict or diagnose neurodegenerative diseases by rapidly measuring specific skin autofluorescence (SAF) in the skin surface in a non-invasive manner, or by measuring the mRNA expression or protein amounts of the biomarker in the biological tissue such as skin, blood, cerebrospinal fluid, and the like.

The neurodegenerative disease is at least one selected from the group consisting of Alzheimer's disease, Parkinson's disease, amyotrophic lateral sclerosis, Huntington's chorea, spinocerebellar degeneration, prion diseases, Creutzfeldt-Jakob disease, frontotemporal dementia, vascular dementia, Lewy body dementia, and dementia with Parkinson's disease.

The kit can diagnose the disease using pentosidine, carboxyethyl lysine (CEL), and carboxymethyl lysine (CML) among the glycotoxins.

Among the proteins bound to glycotoxin, it can be diagnosed using collagen in particular.

Pentosidine, carboxyethyl lysine (CEL), carboxymethyl lysine (CML), and collagen may be used alone or in combination.

Collagen can be used alone or as a complex combined with pentosidine, carboxyethyl lysine (CEL), or carboxymethyl lysine (CML).

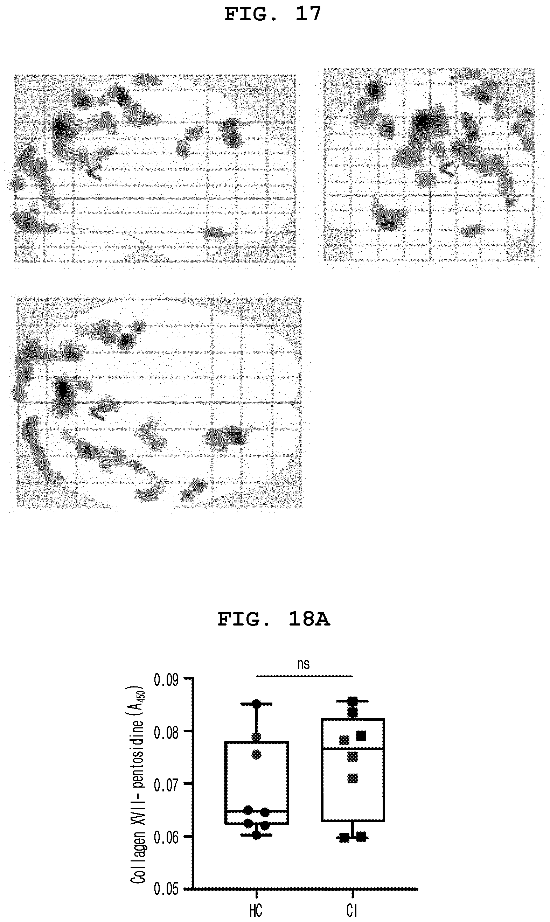

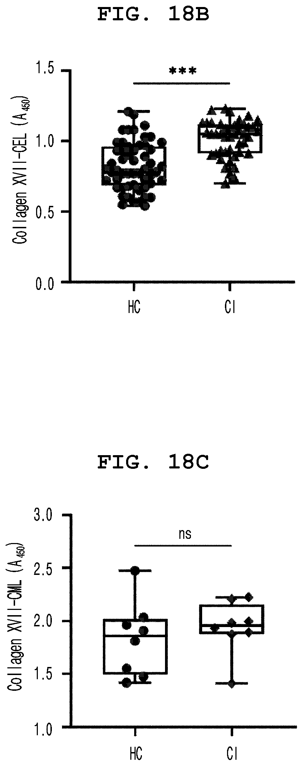

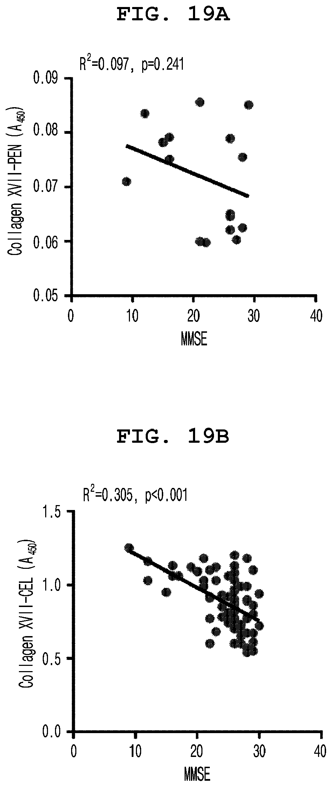

In particular, it was confirmed that CEL bound to collagen XVII was significantly increased (Figures 18 and 19).

Also, CEL/MG-H1+ CEL-COL17A showed more than 90% accuracy.

The SAF values measured in the skin of patients with cognitive decline such as Alzheimer's disease are further increased in the presence of diabetes. Therefore, this SAF can be used to predict the degree of early cognitive decline.

In order to derive key factors of biomarkers with higher selectivity and sensitivity, carboxyethyl lysine (CEL) and methylglyoxal hydroimidazolone (MG-H1), the advanced glycation end-products derived from methylglyoxal in the blood of patients with cognitive decline were measured and the accumulation rates were analyzed.

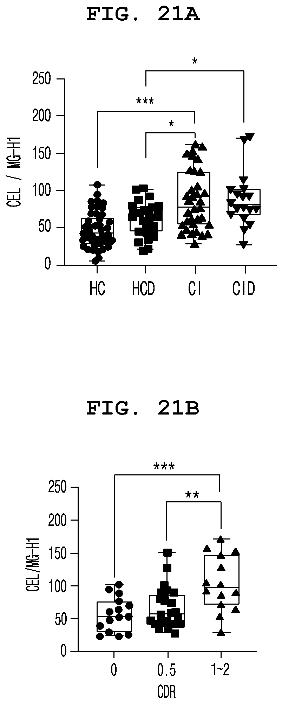

As a result, the ratio of CEL/MG-H1 was increased in the cognitive decline group (CI), diabetic group (HCD), and diabetic cognitive decline group (CID) compared to the normal group (HC), and was particularly increased in the cognitive decline group (CI) and diabetic cognitive decline group (CID) compared to the diabetic group (HCD) (Figure 21a).

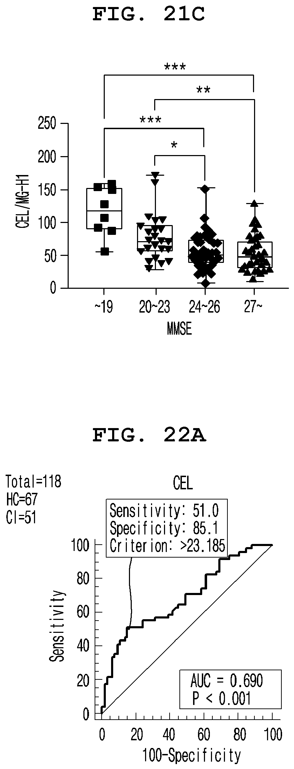

In addition, when the groups were classified according to the severity of Alzheimer's disease through CDR (Clinical Dementia Rating) and MMSE (Mini-Mental State Examination), the CEL/MG-H1 ratio was increased proportionally as the CDR value increased or the MMSE value decreased (Figures 21b and 21c) . This shows that the CEL content in the blood increases and the MG-H1 content decreases in the presence of cognitive decline such as dementia.

Since the values calculated by the CEL/MG-H1 ratio, rather than analyzed by each numerical value of CEL and MG-H1, were significantly different, the present inventor found that the CEL/MG-H1 ratio has very high performance when used for diagnosis and treatment of the above diseases.

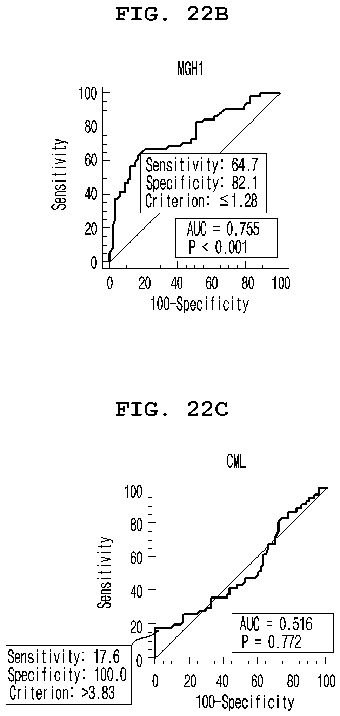

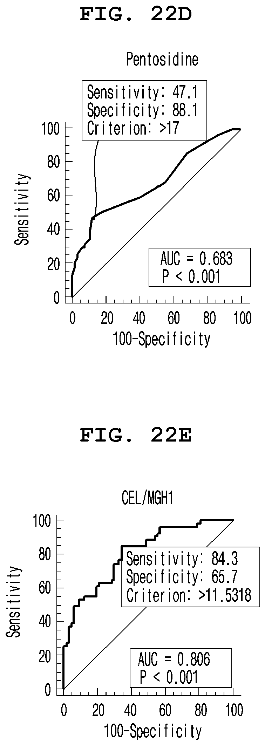

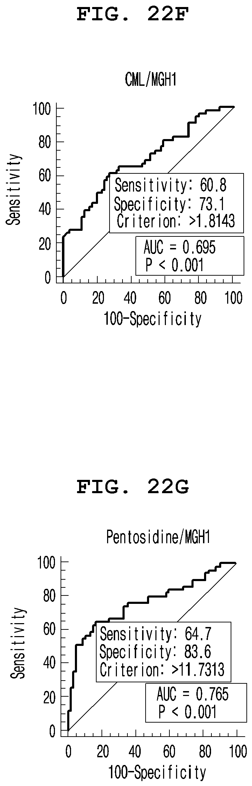

In one experimental example of the present invention, the diagnostic performance of CEL, MG-H1, pentosidine and MG-H1 in the blood of the normal group (HC) and cognitive decline group (CI), and the improvement of diagnostic performance through the combination of each biomarker were analyzed. As a result, the AUC value was significantly increased when the two biomarkers were used in combination than when CEL, MG-H1, or pentosidine was used alone. In this process, CEL/MG-H1 showed the best diagnostic performance. MG-H1/CEL also showed similar diagnostic performance (Figures 22a to 22g).

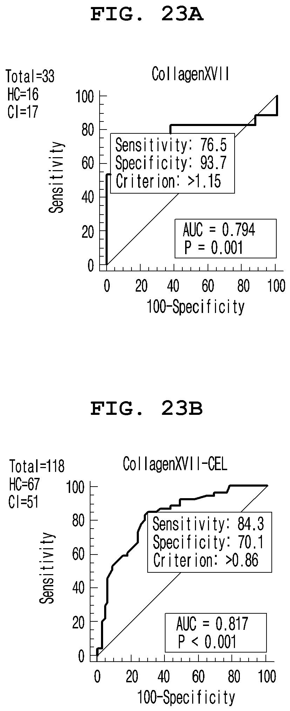

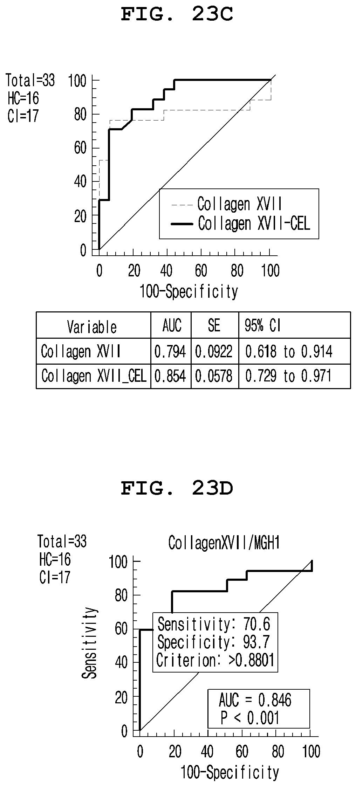

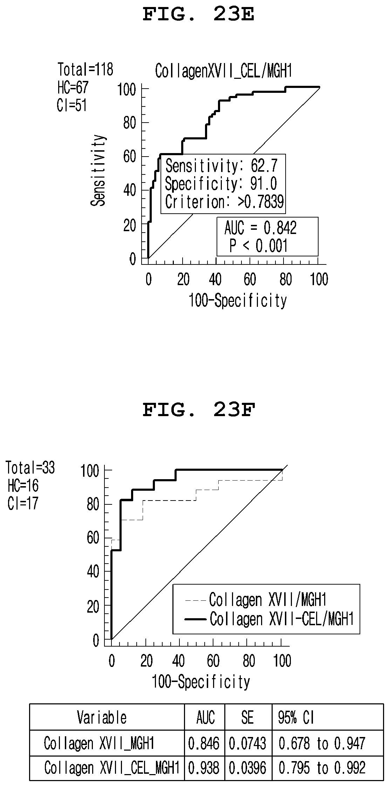

In addition, collagen XVII-CEL was the only single biomarker kit to have AUC value above 0.8, and through this, it can be determined that there is cognitive decline. When MGH1 was combined with other biomarkers as the denominator, CEL/MGH1 and Collagen XVII-CEL/MGH1 had AUC values above 0.8, which suggests cognitive decline (Figures 23a to 23f).

Therefore, when a composite biomarker kit containing both collagen XVII-CEL and MG-H1 was applied, it was confirmed that the diagnostic performance for cognitive decline was excellent.

In particular, the composite biomarker kit shows high accuracy and can be used for the purpose of discriminating the presence or absence of Alzheimer's disease and other systemic physical disorders (depression, chronic disease complications, drug side effects, etc.) that can cause cognitive decline in patients with cognitive decline.

Hereinafter, the present invention will be described in detail by the following experimental examples.

### <Experimental Example 1> Measurement of advanced glycation end-products (AGEs) in plasma and skin tissue of Alzheimer's disease model (5xFAD) mice

Measurement of advanced glycation end-products (AGEs) in the plasma and skin tissue was performed as follows. 5xFAD is an Alzheimer's disease model of 5xFAD transgenic mice expressing Human APP (Swedish mutation: K670N/M671L, Florida mutation: I716V, London mutation: V717I) and PS1 (M146L, L286V) genes. Offspring (wild type, 5xFAD) obtained by breeding B6SJL mice (female) with 5xFAD mice (male) were used. The mice were raised in the controlled condition (light/dark cycle: 12/12 hr, temperature: 22 ± 2°C, and humidity: 55 ± 10%), and allowed to drink water and feed freely (*ad libitum*).

After 12 and 24 weeks, the skin tissues of the normal group (WT) and disease group (5xFAD) mice were ground and treated with a solution (chloroform (Sigma):methanol (Sigma)=2:1) for 12 hours at room temperature (25°C). Collagenase (Sigma C0773) was added thereto at a concentration of 280U, and the mixture was reacted for 24 hours while stirring at 37°C. 2 ul of chloroform and 2 ul of toluene were added thereto to prevent microbial contamination. After adding 1 ml of 6 N HCl to the supernatant and pellet or blood, hydrolysis was performed at 110°C for 24 hours in a heat block. After hydrolysis, the solution was evaporated using a Savant Concentrator, and then 1 ml of distilled water was added thereto. The concentration of collagen in the solution was measured by hydroxyproline assay and using an ELISA kit (STA-675).

Pentosidine in the plasma and skin tissue was measured by HPLC analysis (Table 1) according to the method of Monnier et al. (Monnier. V. et al. Diabetes. 48(4): 870-880. 1999). The amount of collagen was quantified by hydroxyproline assay and then quantified as the amount of pentosidine/collagen. In addition, carboxyethyl lysine (CEL) and carboxymethyl lysine (CML) in the skin hydrolysate were measured using an ELISA kit.

**[Table 1]**

| HPLC conditions for advanced glycation end-products (AGEs) detection | |
|---|---|
| HPLC conditions | |
| Column | Vydac 218TP104 |
| Buffer A | 0.01 mol/L heptafluorobutyric acid |
| Buffer B | 60% acetonitrile (v/v) and 0.01 mol/L heptafluorobutyric acid |
| Detection | 335/385 nm excitation/emission |
| ∼ 10 min | 2% buffer B isocratic |
| 10 ∼ 40 min | 2% to 30% buffer B gradient |
| 40 ∼ 55 min | 30% buffer B isocratic |

As a result, it was confirmed that the amounts of pentosidine, carboxyethyl lysine (CEL), and carboxymethyl lysine (CML) in the skin tissue of the 24-week disease group (5xFAD) were increased significantly (Figures 1a to 1c).

On the other hand, there was no significant difference in the amounts of pentosidine and carboxymethyl lysine (CML) in the plasma of the normal (WT) and the disease group (5xFAD), and the amount of carboxyethyl lysine (CEL) was decreased in both the 12-week and 24-week disease groups (5xFAD) (Figures 2a to 2c).

This means that the advanced glycation end-products (AGEs) accumulate in the skin of patients with Alzheimer's disease (AD), one of the representative neurodegenerative diseases.

Therefore, the advanced glycation end-products accumulated in the skin can be used as biomarkers for diagnosis and treatment of Alzheimer's disease (AD).

### <Experimental Example 2> Measurement of mRNA expression of collagen 1 to 6 and 17 in skin tissue of Alzheimer's disease model (5xFAD) mice

Real-time quantitative RT-PCR was performed to observe gene expression in skin tissue. The skin tissues of the normal group (WT) mice and the Alzheimer's disease model (5xFAD) mice were disrupted and RNA was extracted using Trizol solution (Invitrogen, UK), and total RNA was quantified with NanoDrop ND-1000 (NanoDrop, USA). CDNA was synthesized using reverse transcriptase (TaKaRa, Japan). Primers (Table 2) were added to the synthesized cDNA, and real-time PCR was performed using a real-time PCR machine (ABI Prism 7900HT Sequence Detection SyJPem, Applied BiosyJPems, USA) to measure the gene expression levels of collagen 1 to 6 (Figure 2a) and 17 (Figure 2b).

As a result, the mRNA expression levels of collagen 1, 3, 4 and 5 were decreased in the skin of the disease group (5xFAD), while the mRNA expression levels of collagen 2 and 6 did not show a significant increase (Figures 3a to 3f). On the other hand, it was confirmed that the mRNA expression of collagen XVII in the skin of the disease group (5xFAD) was increased significantly (Figure 4).

This indicates that collagen XVII can be used as a biomarker to diagnose and treat neurodegenerative diseases such as Alzheimer's disease (AD).

**[Table 2]**

| | Primers used for RT-qPCR | | | |
|---|---|---|---|---|
| | SEQ ID NO | Forward sequence (5' -> 3') | SEQ ID NO | Reverse sequence (5' -> 3') |
| *Col1a1* | 1 | CCTCAGGGTATTGCTGGACAAC | 2 | CAGAAGGACCTTGTTTGCCAGG |
| *Col2a1* | 3 | GCTGGTGAAGAAGGCAAACGAG | 4 | CCATCTTGACCTGGGAATCCAC |
| *Col3a1* | 5 | GACCAAAAGGTGATGCTGGACAG | 6 | CAAGACCTCGTGCTCCAGTTAG |
| *Col4a1* | 7 | ATGGCTTGCCTGGAGAGATAGG | 8 | TGGTTGCCCTTTGAGTCCTGGA |
| *Col5a1* | 9 | GGACTCGGCGGAACATT | 10 | GGAGTTGAGGGAACCAAAGAT |
| *Col6a1* | 11 | GACACCTCTCAGTGTGCTCTGT | 12 | GCGATAAGCCTTGGCAGGAAATG |
| *Col17a1* | 13 | GAAAGGAGACAAAGGTGACCA | 14 | CGGCTTGATGGCAATACTTC |
| *Gapdh* | 15 | CCATGGAGAAGGCTGGGG | 16 | CAAAGTTGTCATGGATGACC |

### <Experimental Example 3> Measurement of protein expression level of collagen XVII in skin tissue of Alzheimer's disease model (5xFAD) mice

In order to confirm the protein expression level, the skin tissue was disrupted and dissolved in lysis buffer, followed by centrifugation at 12,000 rpm for 10 minutes to obtain a supernatant. After measuring the protein concentration by the Bradford method, 30 µg of protein was taken and subjected to SDS-PAGE (Sodium Dodecyl Sulfate - PolyAcrylamide Gel Electrophoresis) and then transferred to a nitrocellulose membrane. The membrane was blocked with 5% [w/v] skim milk dissolved in TBS-T (0.05% Tween 20), reacted with antibodies (collagen XVII, α-Tubulin), and then measured.

As a result, it was confirmed that the protein expression of collagen XVII in the skin of the disease group (5xFAD) was significantly increased (Figures 5a and 5b).

This indicates that collagen XVII can be used as a biomarker to diagnose and treat neurodegenerative diseases such as Alzheimer's disease (AD).

### <Experimental Example 4> Measurement of mRNA expression level of collagen XVII in skin of aging mouse model(old)

The mRNA expression level of collagen XVII in the skin of the aging mouse model (old) was measured.

As a result, the mRNA expression level of collagen XVII in the skin was decreased in the aging group (old) compared to the control group (young) (Figure 6).

This indicates that collagen XVII does not increase due to aging, but increases in the presence of Alzheimer's disease (AD).

Therefore, it was confirmed that collagen XVII can be used as a biomarker to diagnose and treat Alzheimer's disease (AD), distinguishing it from aging.

### <Experimental Example 5> Measurement of advanced glycation end-products (AGEs) in plasma of normal group (HC) and cognitive decline group (CI)

Blood was collected from normal individuals and cognitive decline patients, centrifuged to obtain a supernatant, the plasma, and the amounts of pentosidine, carboxyethyl lysine (CEL), and carboxymethyl lysine (CML) in the plasma were measured by HPLC. The HPLC analysis conditions were the same as in Table 1 above.

As a result, the contents of pentosidine, carboxyethyl lysine (CEL) and carboxymethyl lysine (CML) in the plasma of the cognitive decline group (CI) were increased compared to the normal group (HC) (Figures 7a to 7d).

This shows that the contents of pentosidine, carboxyethyl lysine (CEL) and carboxymethyl lysine (CML), the glycotoxins in the plasma increase in the presence of cognitive decline. Therefore, they can be targeted and used for diagnosis and treatment of the disease.

### <Experimental Example 6> Measurement of collagen XVII content in plasma of normal group (HC) and cognitive decline group (CI)

Blood was collected from normal individuals and cognitive decline patients, centrifuged to obtain a supernatant, the plasma, and the content of collagen XVII in the plasma was measured using human collagen XVII ELISA kit (CSB-EL005724HU). This experiment was conducted according to the manufacturer's protocol.

As a result, it was confirmed that collagen XVII in the blood was significantly increased in the cognitive decline group (CI) compared to the normal group (HC) (Figure 8).

This shows that the content of collagen XVII in the blood increases in the presence of cognitive decline. Therefore, it can be targeted and used for diagnosis and treatment of cognitive decline.

### <Experimental Example 7> Measurement of glyoxalase 1 enzyme activity in blood of normal group (HC) and cognitive decline group (CI)

Glyoxalase (GLO) is a protein directly involved in the metabolism of methylglyoxal (MG). Methylglyoxal is a precursor of CEL and is finally converted to D-lactate by the GLO enzyme.

Blood was collected from normal individuals and cognitive decline patients, centrifuged to obtain a supernatant, the plasma, and the enzyme activity of glyoxalase 1 in the blood was measured by ELISA assay (QuantiChrom^{™}, DGLO-100).

As a result, the activity of glyoxalase 1 in the plasma of the cognitive decline group (CI) was lower than that in the plasma of the normal group (HC) (Figures 9a and 9b).

The fact that the activity of glyoxalase 1, a methylglyoxal lyase, is lower in patients with cognitive decline indicates that methylglyoxal, a precursor of carboxyethyl lysine (CEL), is less degraded.

This indicates that the less degraded methylglyoxal in patients with cognitive decline can be converted to carboxyethyl lysine (CEL) more, and consequently, the amount of carboxyethyl lysine can be increased in patients with cognitive decline. These results are consistent with the increase of carboxyethyl lysine (CEL) in the plasma of the cognitive decline group (CI), the results of Experimental Example 5.

### <Experimental Example 8> Comparison of correlation between cognitive decline and biomarker of present invention through novel object recognition test (NORT) on Alzheimer's disease model (5xFAD) mice

A novel object recognition test (NORT) was performed on Alzheimer's disease model (5xFAD) mice. For the test, the mice (5xFAD) were transferred to a behavioral observation room and acclimatized for 5 minutes. In the 3-minute exposure trial, 2 identical objects (1 set) were placed in a box 30 cm apart, and the time for the animal to explore the objects for 3 minutes was measured. In the withdrawal trial one day later, the same box was placed with one object revealed during exposure and one newly substituted object, and the animal's exploration time was measured.

The exploration time was calculated as (time showing interest in new objects - time showing interest in familiar objects) / (time showing interest in new objects + time showing interest in familiar objects). The calculated value was represented as a recognition index, and the lower the value, the lower the cognitive ability.

As a result, as the cognitive ability of the mice decreased, the content of pentosidine, CEL, and CML in the skin tended to increase, among which pentosidine (*p* = 0.0133) and CEL (*p* = 0.008) were highly correlated with the cognitive ability. On the other hand, CML (*p* = 0.1166) showed a low correlation with the cognitive ability (Figures 10a to 10c).

In addition, the mRNA expression of collagen XVII in the skin tended to increase as the cognitive function decreased (Figure 11).

Accordingly, it was confirmed that there is a correlation between cognitive decline and pentosidine, carboxyethyl lysine (CEL), and collagen XVII.

### <Experimental Example 9> Measurement of advanced glycation end-products bound to collagen XVII in blood of patients with cognitive decline

### <Experimental Example 9-1> Amount of advanced glycation end-products bound to collagen XVII in plasma of normal group (HC) and cognitive decline group (CI)

Blood was collected from normal individuals and cognitive decline patients, centrifuged to obtain a supernatant, the plasma, and the amount of advanced glycation end-products bound to collagen XVII in the blood was measured using a human collagen XVII ELISA kit (CSB-EL005724HU) with pentosidine, CEL and CML antibodies. In this experiment, the blood was incubated with a plate coated with a collagen XVII capture antibody to bind the collagen XVII in the blood to the capture antibody. Then, the fixed collagen XVII was bound to the detection antibodies such as pentosidine, CEL, and CML antibodies to induce a substrate enzyme reaction to generate a color change, which was measured as an optical density value.

As a result, it was confirmed that CEL bound to collagen XVII in the blood of the cognitive decline group (CI) was significantly increased compared to the normal group (HC) (Figures 18a to 18c and Figures 19a to 19c).

### <Experimental Example 9-2> Expression patterns of collagen XVII and CEL in plasma of cognitive decline group (CI)

In addition, the presence of collagen XVII protein and CEL in the blood was confirmed by Western blotting.

Blood proteins were loaded into a gel made of 8% acryl amide and electrophoresed at 100 volts for 1 hour. After electrophoresis, a membrane was placed on the gel into which the sample was loaded, and electrophoresis was performed at 4°C for 1 hour at 80 volts. The membrane on which the sample was transferred was washed with PBS, blocked with 5% skim milk for 1 hour at room temperature, and then washed three times with PBS for 10 minutes each. Thereafter, the membrane was immersed in a mixture of anticollagen XVII antibody and anti-CEL antibody, the primary antibodies, in 5% skim milk, and left at 4°C for 12 hours. After 12 hours, the membrane was washed three times with PBS for 10 minutes each and then incubated in a mixture of anti-rabbit and anti-mouse antibodies, the secondary antibodies, in 5% skim milk for 1 hour at room temperature. The membrane was washed again three times for 10 minutes each, reacted with horseradish peroxidase, and then displayed on a film.

As a result, collagen XVII and CEL were detected at the same protein size position (~70 kDa) among several protein size positions, indicating that when CEL is present in blood, it binds to collagen XVII to form advanced glycation end-products (Figure 20).

This shows that the increased content of collagen XVII in the blood is selectively bound to CEL rather than other advanced glycation end-products in the presence of cognitive decline such as dementia. Therefore, it can be targeted and used for prediction and diagnosis of the disease.

### <Experimental Example 10> Measurement of skin autofluorescence (SAF) specific to biomarker of present invention through noninvasive method

### <Experimental Example 10-1> Skin autofluorescence (SAF) of normal group (HC), diabetic group (HCD), cognitive decline group (CI) and diabetic cognitive decline group (CID)

Skin autofluorescence (SAF) was measured by an AGE Reader (DiagnoOptics Technologies BV) capable of detecting the fluorescence signals of specific advanced glycation end-products (AGEs), and all measurements were performed on the volar side of the forearm between 10 and 15 cm below the elbow. Through this, it was possible to measure the level of advanced glycation end-products (AGEs) accumulated in 1 cm² of the skin surface.

The measurement subjects were the normal group (HC), diabetic group (HCD), cognitive decline group (CI) and diabetic cognitive decline group (CID), and the measured values were calculated by dividing the mean value of the emitted light intensity per nm between 420 and 600 nm by the mean value of the excitation light intensity per nm between 300 and 420 nm, and expressed in arbitrary units (AU).

As a result, skin autofluorescence (SAF) was increased in the diabetic cognitive decline group (CID), cognitive decline group (CI), and diabetic group (HCD) compared to the normal group (HC), and increased further in the diabetic cognitive decline group (CID) group compared to the cognitive decline group (CI) and diabetic group (HCD) (CID vs CI, P= 0.019; CID vs HCD, *P*= 0.000)(Figure 13).

An increase in the measured value of skin autofluorescence (SAF) can be a major indicator of diabetes, and in the present invention, it was confirmed that the measured value of skin autofluorescence (SAF) was increased even in the cognitive decline group (CI) without diabetes. This means that the measured value of skin autofluorescence (SAF) is associated with cognitive decline independent of diabetes. In particular, the group with both diabetes and cognitive decline (CID) showed the highest value of skin autofluorescence (SAF), which can be interpreted as the interaction of diabetes and cognitive decline.

The SAF value was increased to a high level in the cognitive decline group (CI) without diabetes, indicating that glycotoxins in the skin could be breakthrough markers for neurodegenerative diseases including Alzheimer's disease. Furthermore, the highest SAF values were found in the diabetic group with cognitive decline, suggesting that glycotoxins in the skin of patients with diabetes can be used for diagnosis of neurodegenerative diseases.

### <Experimental Example 10-2> Measurement of diagnostic performance through skin autofluorescence between normal group (HC + HCD) and cognitive decline group (CI + CID)

ROC (Receiver Operating Characteristic) curve analysis was performed to determine whether the skin autofluorescence levels could distinguish between the normal group (HC + HCD) and the cognitive decline group (CI + CID). The ROC curve analysis is used to determine the usefulness of a test, evaluate the accuracy of a test, or set a cut-off point for diagnosis.

As a result, the AUC value was 0.763 (the closer the value is to 1, the higher the diagnostic performance), showing a satisfactory level of diagnostic performance, and the sensitivity and specificity were 68.1% and 76.7%, respectively (Figure 14) .

Since the above analysis method can confirm cognitive decline within only 12 seconds, the AUC value can be used as an index that can quickly and non-invasively identify patients with cognitive decline. In particular, it can be sufficiently used to diagnose dementia independently of diabetes.

### <Experimental Example 10-3> Correlation between skin autofluorescence (SAF) and clinical indicators in non-diabetic group

The association of SAF with other clinical variables was investigated in two groups divided according to the presence or absence of diabetes.

Correlation of skin autofluorescence (SAF) with CDR (Clinical Dementia Rating), MMSE (Mini-Mental State Examination), and blood test results (glucose, HbA1c, total cholesterol, triacylglycerol, HDL, LDL, creatinine and CKD_EPI levels) in non-diabetic subjects (HC and CI) was analyzed.

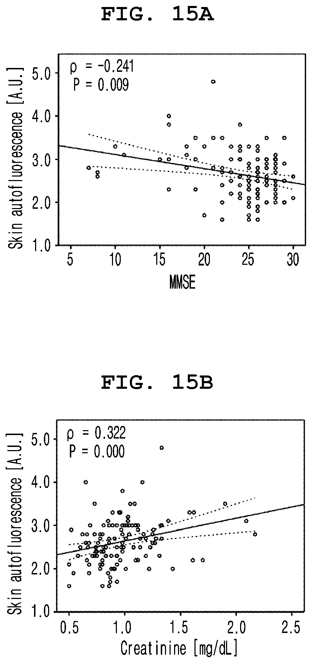

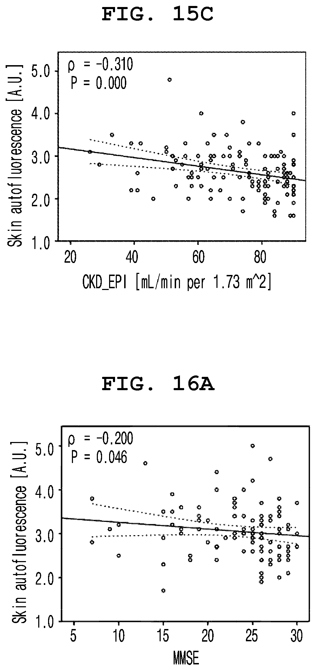

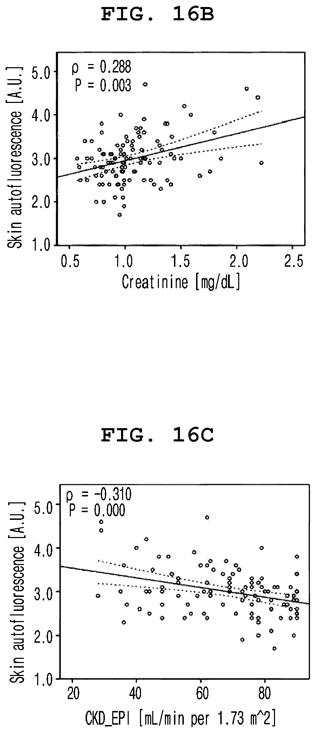

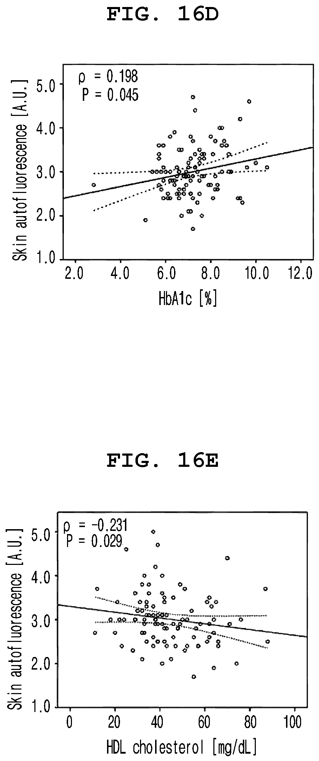

As a result, skin autofluorescence (SAF) was highly correlated with cognitive decline (MMSE, ρ = - 0.241, *P* = 0.009) and renal function markers (creatinine: ρ = 0.322, *P* = 0.000 and CKD-EPI: ρ = - 0.310, *P* = 0.000) in non-diabetic subjects (HC and CI). On the other hand, there was no correlation with HbA1c (β = -0.120, P = 0.281), a representative indicator of diabetes, among the blood test results (Figures 15 and 16).

**[Table 3]**

| | Non-DM | | | DM | | |
|---|---|---|---|---|---|---|
| | HC (n=71 ) | CI (n=55 ) | *P value* | HCD (n=45 ) | CID (n=64 ) | *P value* |
| Age | 75 [73-77] | 76 [73-77] | 0.159^{a} | 75 [72-76] | 75 [72-77] | 0.385^{a} |
| Gender (male/female), n | 56/15 | 46/9 | 0.499^{b} | 37/8 | 57/7 | 0.307^{b} |
| MMSE | 26 [25-28] | 23 [19-26] | 0.000^{a} | 27 [26-28] | 22 [17-25] | 0.000^{a} |
| CDR, 0/0.5/1/2/3 | 52/19 /0/0/ 0 | 0/30/ 16/7/ 2 | 0.000^{c} | 35/10 /0/0/ 0 | 0/29/ 26/8/ 1 | 0.000^{c} |
| APOE4 (-/+), n | 29/11 | 26/11 | 0.829^{b} | 13/3 | 27/13 | 0.304^{b} |
| Education, year | 9 [6-12] | 10 [6-12] | 0.851^{a} | 9 [7-12] | 10 [7-12] | 0.484^{a} |
| Current smoker, 0/1/2 | 54/10 /7 | 33/14 /8 | 0.147^{b} | 33/7/ 5 | 37/24 /3 | 0.030^{c} |
| Hypertension, n | 45 (64.3 o) | 34 (63.0 o) | 0.879^{b} | 37 (82.2 o) | 53 (82.8 o) | 0.936^{b} |
| Coronary heart Disease, n | 10 (14.3 o) | 7 (14.0 o) | 0.965^{b} | 9 (20.0 o) | 12 (18.8 o) | 0.871^{b} |
| Atrial fibrillation, n | 3 (4.3 o) | 3 (6.1 o) | 0.689^{c} | 3 (6.7 o) | 6 (9.4 o) | 0.734^{c} |
| Angina pectoris, n | 14 (20.3 %) | 11 (21.6 %) | 0.865^{b} | 11 (24.4 %) | 12 (18.8 %) | 0.473^{b} |
| Chronic kidney disease, n | 16 (22.5 %) | 14 (29.4 %) | 0.389^{b} | 17 (37.8 %) | 24 (39.3 %) | 0.870^{b} |
| Glucose, mg/dL | 102 [94-112] | 103 [93-119] | 0.715^{a} | 131 [111-161] | 129 [115-174] | 0.811^{a} |
| HbA_{1c}, % | 5.7 [5.5-6.1] | 5.8 [5.5-6.1] | 0.526^{a} | 7.1 [6.3-8.0] | 7.1 [6.5-8.0] | 0.871^{a} |
| Total cholesterol, mg/dL | 162 ± 33 | 161 ± 36 | 0.861^{d} | 145 ± 24 | 149 ± 43 | 0.610^{d} |
| Triacylglycerol, mg/dL | 99 [76-134] | 109 [85-145] | 0.451^{a} | 139 [88-192] | 137 [89-192] | 0.839^{a} |
| HDL cholesterol, mg/dL | 55 [44-65] | 51 [43-67] | 0.950^{a} | 43 [37-61] | 41 [34-54] | 0.361^{a} |
| LDL cholesterol, mg/dL | 94 [75-113] | 92 [68-110] | 0.816^{a} | 81 [71-91] | 80 [63-97] | 0.934^{a} |
| Creatinine, | 0.9 | 1.0 | 0.012^{a} | 1.0 | 1.0 | 0.883^{a} |
| mg/dL | [0.8-1.1] | [0.9-1.2] | | [0.9-1.3] | [0.9-1.2] | |
| CKD-EPI, mL/min per 1.73 m² | 81 [64-88] | 70 [56-84] | 0.036^{a} | 72 [54-82] | 73 [58-83] | 0.883^{a} |
| SAF | 2.4 [2.2-2.7] | 3.0 [2.6-3.3] | 0.000^{a} | 2.8 [2.5-3.1] | 3.1 [2.9-3.5] | 0.000^{a} |

From the data showing high correlation with MMSE, creatinine, and CKD-EPI and no correlation with HbA1c, it was confirmed that the results of skin autofluorescence (SAF) in non-diabetic subjects (HC and CI) were specific for cognitive decline and independent of diabetes (Table 3).

### <Experimental Example 10-4> Correlation between skin autofluorescence (SAF) and clinical indicators in diabetic group

Correlation of skin autofluorescence (SAF) with MMSE (Mini-Mental State Examination), and blood test results (glucose, HbA1c, total cholesterol, triacylglycerol, HDL, LDL, creatinine and CKD_EPI levels) in diabetic subjects (HCD and CID) was analyzed.

As a result, skin autofluorescence (SAF) was highly correlated with cognitive decline (MMSE, ρ = - 0.241, *P* = 0.009) in non-diabetic subjects (HC and CI). Among the blood test results, it was highly correlated with creatinine (ρ = 0.322, P = 0.000 and CKD-EPI: ρ = -0.310, P = 0.000), an indicator of kidney function, and did not correlate with HbA1c and HDL, typical indicators of diabetes.

As a result, the correlation between cognitive decline (MMSE, ρ = -0.200, P = 0.046) and skin autofluorescence (SAF) in subjects with diabetes (HCD and CID) was lower than the correlation between cognitive decline and skin autofluorescence (SAF) in subjects without diabetes (HC and CI). On the other hand, skin autofluorescence was highly correlated with creatinine (Disease = 0.288, P = 0.003 and CKD-EPI: ρ = - 0.310, P = 0.000), a kidney function indicator, and poorly correlated with HbA1c (ρ = 0.198, P = 0.045) and HDL (ρ = -0.231, P = 0.029), representative indicators of diabetes (Figures 16a to 16e).

In non-diabetic subjects (HC and CI), skin autofluorescence (SAF) was correlated with cognitive decline rather than HbA1c and HDL, suggesting that skin autofluorescence (SAF) was increased in these groups through a mechanism independent of diabetes.

In diabetic subjects (HCD and CID), skin autofluorescence (SAF) was correlated with diabetes rather than cognitive decline, suggesting that skin autofluorescence (SAF) was increased in these groups through a mechanism related to diabetes. In addition, in subjects with both diabetes and cognitive decline (CID), it was confirmed that skin autofluorescence (SAF) was affected by both aspects of diabetes and cognitive decline, with diabetes having a greater effect.

### <Experimental Example 11> Analysis of variables affecting cognitive decline

In order to analyze the variables affecting cognitive decline, correlations between skin autofluorescence levels, various clinical indicators, and blood test results were analyzed. In the univariate model, the variables affecting cognitive decline were analyzed separately, and in the multivariate model, all indicators were included and the influence of each variable was considered, and then the variables affecting cognitive decline were identified.

As a result, in the univariate model, it was confirmed that skin autofluorescence, diabetes, and glucose each had an effect on cognitive decline. Among them, skin autofluorescence had the highest odds ratio of 6.711. In the multivariate analysis with clinical indicators, it was found that skin autofluorescence and smoking status had an effect on cognitive decline. When analyzed with the addition of blood tests, SAF, smoking status, and total cholesterol had an effect on cognitive decline (Table 4).

In all analysis results, it was confirmed that the skin autofluorescence values have the greatest influence on cognitive decline. Through these results, it was once again verified that skin autofluorescence is a factor that plays an important role in cognitive decline.

**[Table 4]**

| **Analysis** | **Odds ratio (95% CI)** | ***P value*** |
|---|---|---|
| Univariate model | | |
| Age | 1.027 (0.964-1.095) | 0.406 |
| Male | 1.592 (0.793-3.196) | 0.191 |
| Education | 1.000 (0.935-1.069) | 0.994 |
| Current smoker | 2.657 (0.867-8.145) | 0.084 |
| SAF | 6.711 (3.580-12.580) | **0.000** |
| DM | 1.836 (1.093-3.085) | **0.022** |
| Hypertension | 1.129 (0.635-2.008) | 0.679 |
| Coronary heart Disease | 1.011 (0.504-2.027) | 0.976 |
| Chronic kidney disease | 1.344 (0.767-2.353) | 0.301 |
| Angina pectoris | 0.890 (0.471-1.683) | 0.720 |
| Atrial fibrillation | 1.572 (0.541-4.571) | 0.406 |
| Glucose | 1.008 (1.001-1.015) | **0.031** |
| HbA_{1c} | 1.269 (0.990-1.625) | 0.060 |
| Creatinine | 2.208 (0.925-5.272) | 0.074 |
| CKD-EPI | 0.986 (0.970-1.002) | 0.083 |
| Total cholesterol | 0.999 (0.991-1.007) | 0.816 |
| HDL | 0.987 (0.968-1.006) | 0.164 |
| LDL | 1.002 (0.996-1.009) | 0.527 |
| Triacylglycerol | 1.003 (0.999-1.007) | 0.165 |

| Multivariate model 1^{a} | | |
|---|---|---|
| SAF | 6.238 (3.256-11.949) | **0.000** |
| Current smoker | 2.755 (0.925-8.204) | 0.069 |

| Multivariate model 2^{b} | | |
|---|---|---|
| SAF | 8.420 (3.415-20.763) | **0.000** |
| Current smoker | 3.750 (0.959-14.663) | 0.057 |
| Total cholesterol | 1.014 (1.002-1.025) | **0.017** |

Age, education, SAF, glucose, HbA1c, creatinine, CKD-EPI, total cholesterol, triacylglycerol, HDL cholesterol, and LDL cholesterol were defined as continuous variables. Male, current smoker, DM, hypertension, coronary heart disease, chronic kidney disease, angina pectoris, and atrial fibrillation were defined as categorical variables.

^{a}Age, male, education, current smoker, SAF, DM, angina pectoris, coronary heart disease, chronic kidney disease, and atrial fibrillation were included as variates.

^{b}Age, male, education, current smoker, SAF, glucose, HbA1c, creatinine, CKD-EPI, total cholesterol, HDL, LDL, and triacylglycerols were included as variates.

### <Experimental Example 12> Analysis of odds ratio of skin autofluorescence and MRI results

The correlation between skin autofluorescence and MRI (magnetic resonance imaging) results was analyzed. All indicates the analysis results including all groups (HC, HCD, CI, and CID). According to DM (diabetes mellitus), the analysis was performed by dividing into non-DM (HC+CI) and DM (HCD+CID).

As a result, when analyzed in all groups, significant statistical values were shown in all results except for periventricular white matter hyperintensities (PVWMH). Interestingly, when analyzed by dividing into non-DM and DM, they exhibit distinct patterns. In non-DM groups, both hippocampal atrophy (HA) and parietal atrophy (PA) showed strong statistical significance. In DM groups, it was confirmed that the correlation between PA and skin autofluorescence was weakened. In the case of Fazekas scale, the correlation was stronger in DM than in non-DM (Table 5).

These results show that skin autofluorescence is sufficiently reflective of substantial changes in the brain. In all experimental groups, skin autofluorescence showed a high correlation with brain atrophy and white matter hyperintensity. In addition, skin autofluorescence reflected brain changes differently depending on the presence or absence of diabetes.

Skin autofluorescence largely reflected atrophy in non-DM, while it was less reflective of atrophy and more correlated with WMH in DM.

**[Table 5]**

| | All^{a} | | non-DM^{b} | | DM^{b} | |
|---|---|---|---|---|---|---|
| | Odds ratio (95% CI) | *P* | Odds ratio (95% CI) | *P* | Odds ratio (95% CI) | *P* |
| Left hippocampal atrophy(HA) | 1.772 (1.176-2.672) | **0.006** | 2.004 (1.130-3.695) | **0.018** | 1.536 (0.866-2.724) | 0.142 |
| Right hippocampal atrophy(HA) | 1.738 (1.138-2.656) | **0.011** | 1.824 (0.987-3.370) | 0.055 | 1.680 (0.930-3.037) | 0.086 |
| Left parietal atrophy(PA) | 2.212 (1.390-3.522) | **0.001** | 2.641 (1.344-5.181) | **0.005** | 2.012 (1.046-3.869) | **0.036** |
| Right parietal atrophy(PA) | 2.175 (1.366-3.459) | **0.001** | 2.641 (1.344-5.181) | **0.005** | 1.935 (1.009-3.706) | **0.047** |
| PVWMH | 1.455 (0.917-2.309) | 0.111 | 1.567 (0.811-3.019) | 0.179 | 1.445 (0.740-2.821) | 0.281 |
| DWMH | 1.809 (1.055-3.102) | **0.031** | 1.815 (0.850-3.877) | 0.124 | 1.970 (0.911-4.259) | 0.085 |
| Fazekas scale | 1.632 (1.044-2.552) | **0.032** | 1.255 (0.705-2.469) | 0.386 | 2.168 (1.131-4.154) | **0.020** |

| | | | | | | |
|---|---|---|---|---|---|---|
| ^{a}Age, gender, SAF, and DM were included as variates. ^{b}Age, gender, and SAF were included as variates. | | | | | | |

### <Experimental Example 13> Analysis of correlation between skin autofluorescence and FDG-PET

The correlation between FDG(18F-fludeoxyglucose)-PET(positron emission tomography) showing brain glucose metabolism and skin autofluorescence in the Non-DM(HC+CI) group was visualized. The DM group was excluded from this analysis because it had an abnormal effect on FDG-PET.

The part correlated with the increase in autofluorescence and the decrease in glucose metabolism was indicated by the difference in contrast, and the darker the color, the higher the correlation. As shown in Figure 17, the correlation between the decreased brain glucose metabolism and the increased skin autofluorescence was strongest in the parietal cortex and the posterior cingulate gyrus.

The metabolic reduction in the parietal cortex coincided with the atrophy results on MRI (magnetic resonance imaging), confirming once again that skin autofluorescence indicates changes in the brain. In particular, the posterior cingulate gyrus is known to be the most important region for diagnosing Alzheimer's disease in FDG-PET. These results indicate that the increase in skin autofluorescence affects the onset of brain lesions in the parietal cortex and posterior cingulate gyrus, leading to a decrease in glucose metabolism. In addition, the correlation between skin autofluorescence and decreased glucose metabolism in the posterior cingulate gyrus suggests that skin autofluorescence reflects the changes in the brain caused by Alzheimer's disease (Figure 17).

### <Experimental Example 14> Analysis of accumulation ratio of carboxyethyl lysine (CEL) and methylglyoxal hydroimidazolone (MG-H1), advanced glycation end-products derived from methylglyoxal, in the blood of patients with cognitive decline

Blood was collected from normal individuals and cognitive decline patients, centrifuged to obtain a supernatant, the plasma, and the amounts of CEL and MG-H1 in the blood were measured by ELISA. This experiment was conducted according to the manufacturer's protocol. Then, the ratio of each advanced glycation end-product accumulated in the blood was compared and shown.

As a result, the ratio of CEL/MG-H1 was increased in the cognitive decline group (CI), diabetic group (HCD), and diabetic cognitive decline group (CID) compared to the normal group (HC), and was particularly increased in the cognitive decline group (CI) and diabetic cognitive decline group (CID) compared to the diabetic group (HCD) (Figure 21a).

In addition, when the groups were classified according to the severity of Alzheimer's disease through CDR (Clinical Dementia Rating) and MMSE (Mini-Mental State Examination), the CEL/MG-H1 ratio was increased proportionally as the CDR value increased or the MMSE value decreased (Figures 21b and 21c).

This shows a similar trend to the results of Experimental Example 7 (Figures 9a and 9b), indicating that the CEL content in the blood increases and the MG-H1 content decreases in the presence of cognitive decline such as dementia. Therefore, CEL, MG-H1, and CEL/MG-H1 can be targeted and used for diagnosis and treatment of the disease.

### <Experimental Example 15> Analysis of accumulation ratio of collagen XVII-CEL, CEL/MG-H1, and MG-H1/CEL in blood of patients with cognitive decline

### <Experimental Example 15-1> Analysis of accumulation ratio of collagen XVII-CEL, CEL/MG-H1 and MG-H1/CEL in blood of normal group (HC) and cognitive decline group (CI)

To evaluate the accuracy of diagnostic values of biomarkers, AUC (Area under the Curve) was analyzed in ROC (Receiver Operator Characteristic) curve. The cutoff values were obtained according to the sensitivity and specificity of the point where the Youden index was maximized. Since the number of samples measured for each biomarker was different, they were analyzed independently.

As a result, the AUC values of each CEL, MG-H1, CML, and pentosidine biomarkers were 0.690, 0.755, 0.516, and 0.683, respectively, but the composite biomarker kit with CEL/MG-H1 showed the highest accuracy with the AUC value of 0.806, and the sensitivity and specificity were 84.3% and 65.7%, respectively, at a cutoff of 11.53. CML/MG-H1 and pentosidine/MG-H1 showed slightly lower diagnostic performance than CEL/MG-H1 with the AUC values of 0.695 and 0.765. For cutoffs of 1.81 and 11.73, the sensitivities were 60.8 and 64.7%, and the accuracies were 73.1 and 83.6% (Figures 22a to 22f).

The above results were based on the ROC curve analysis, and the cutoff was selected as the point with the best sensitivity and specificity. When the cut-off of the composite biomarker kit with CEL/MG-H1 was set at 11.53, and a higher value was judged as cognitive decline and a lower value as normal, the sensitivity and accuracy were 84.3% and 65.7%, respectively. Since the cutoff value of 11.53 showed the highest sensitivity and accuracy than other cutoff values, it can be judged as cognitive decline if the value is higher than 11.53 based on this.

In addition, collagen XVII-CEL was the only single biomarker to exhibit an AUC value above 0.8. When the cut-off value was set at 0.86, and a higher value was judged as cognitive decline and a lower value as normal, the sensitivity and accuracy were 84.3% and 65.7%, respectively. Since the cutoff value of 0.86 showed the highest sensitivity and accuracy than other cutoff values, it can be judged as cognitive decline if the value is higher than 0.86 based on this.

When MGH1 was combined with other biomarkers as the denominator, the AUC values of CEL/MGH1 and Collagen XVII-CEL/MGH1 exceeded 0.8. Thus, when the cutoff was set at 0.7839 and a higher value was judged as cognitive decline and a lower value as normal, the sensitivity and accuracy were 70.6% and 93.7%, respectively. Since the cutoff value of 0.7839 showed the highest sensitivity and accuracy than other cutoff values, it can be judged as cognitive decline if the value is higher than 0.7839 based on this (Figures 23a to 23f).

Therefore, when a composite biomarker kit containing both collagen XVII-CEL and MG-H1 was applied, it was confirmed that the diagnostic performance for cognitive decline was excellent.

## Claims

1. A biomarker for predicting or diagnosing neurodegenerative diseases, comprising glycotoxin, a specific protein bound to glycotoxin, or a glycotoxin-specific protein complex.

2. The biomarker for predicting or diagnosing neurodegenerative diseases according to claim 1, wherein the glycotoxin is at least one selected from the group consisting of pentosidine, carboxymethyl lysine (CML), carboxyethyl lysine (CEL), pyrraline, OMA (oxalic acid monolysinylamide), imidazolones, GLAP (glyceraldehydes-derived pyridinum compound), GOLD (glyoxal-lysine dimer), MOLD (methyl-glyoxal-lysine dimmer), crossline, and FFI (2-(2-furoyl)-4(5)-(2-furanyl)-1H-imidazole).

3. The biomarker for predicting or diagnosing neurodegenerative diseases according to claim 2, wherein the biomarker is used for measuring at least one glycotoxin selected from the group consisting of pentosidine, carboxyethyl lysine (CEL), carboxymethyllysine (CML), and methylglyoxal hydroimidazolone (MG-H1).

4. The biomarker for predicting or diagnosing neurodegenerative diseases according to claim 1, wherein the specific protein is at least one selected from the group consisting of collagen, elastin, keratin, amyloid β species, tau, alpha-synuclein, and TDP-43.

5. The biomarker for predicting or diagnosing neurodegenerative diseases according to claim 4, wherein the collagen is collagen XVII.

6. The biomarker for predicting or diagnosing neurodegenerative diseases according to claim 1, wherein the neurodegenerative disease is at least one selected from the group consisting of Alzheimer's disease, Parkinson's disease, amyotrophic lateral sclerosis, Huntington's chorea, spinocerebellar degeneration, prion diseases, Creutzfeldt-Jakob disease, frontotemporal dementia, vascular dementia, Lewy body dementia, and dementia with Parkinson's disease.

7. A method for predicting or diagnosing neurodegenerative diseases comprising the following steps:
a step of preparing a sample for measurement from biological tissue or biological fluid; and
a step of measuring glycotoxin, a specific protein bound to glycotoxin, or a glycotoxin-specific protein complex in the sample for measurement.

8. The method for predicting or diagnosing neurodegenerative diseases according to claim 7, wherein the glycotoxin, the specific protein bound to glycotoxin, or the glycotoxin-specific protein complex is measured by any one or more methods of measuring fluorescence, gene expression level, and protein amount.

9. The method for predicting or diagnosing neurodegenerative diseases according to claim 7, wherein the measuring glycotoxin, a specific protein bound to glycotoxin, or a glycotoxin-specific protein complex is to measure at least one selected from the group consisting of pentosidine, carboxyethyl lysine (CEL), carboxymethyl lysine (CML), and methylglyoxal hydroimidazolone (MG-H1).

10. The method for predicting or diagnosing neurodegenerative diseases according to claim 7, wherein the specific protein bound to glycotoxin is collagen.

11. The method for predicting or diagnosing neurodegenerative diseases according to claim 7, wherein the measuring glycotoxin, a specific protein bound to glycotoxin, or a glycotoxin-specific protein complex is to measure a complex of pentosidine, carboxyethyl lysine (CEL) or carboxymethyl lysine (CML) with collagenXVII.

12. A method for predicting or diagnosing neurodegenerative diseases by measuring the accumulation ratio of the glycotoxin, the specific protein bound to glycotoxin, or the glycotoxin-specific protein complex of claim 1, and methylglyoxal hydroimidazolone (MG-H1).

13. The method for predicting or diagnosing neurodegenerative diseases according to claim 12, wherein the measuring the accumulation ratio is to measure at least one selected from the group consisting of CEL/MGH1, CML/MGH1, pentosidine/MGH1, collagen XVII/MGH1, collagen XVII-CEL/ MGH1, collagen XVII-CML/MGH1 and collagen XVII-pentosidine/MGH1.

14. The method for predicting or diagnosing neurodegenerative diseases according to claim 12, wherein the measuring the accumulation ratio is to measure [any one of CEL/MGH1, CML/MGH1, pentosidine/MGH1, or collagen XVII/MGH1] and [a complex of collagen XVII with at least one selected from the group consisting of pentosidine, carboxyethyl lysine (CEL), and carboxymethyl lysine (CML)].

15. The method for predicting or diagnosing neurodegenerative diseases according to claim 12, wherein the accumulation ratio is CEL/MGH1 or collagen XVII-CEL.

16. The method for predicting or diagnosing neurodegenerative diseases according to claim 7, wherein the biological tissue is skin, fingernails, toenails, or hair.

17. The method for predicting or diagnosing neurodegenerative diseases according to claim 7, wherein the biological fluid is tears, saliva, urine, blood, or cerebrospinal fluid.

18. The method for predicting or diagnosing neurodegenerative diseases according to claim 7, wherein the measuring the biological tissue is to measure skin autofluorescence (SAF) that does not require a sample for measurement non-invasively.

19. A kit for predicting or diagnosing neurodegenerative diseases that measures the glycotoxin, the specific protein bound to glycotoxin, or the glycotoxin-specific protein complex of claim 1.

20. The kit for predicting or diagnosing neurodegenerative diseases according to claim 19, wherein the kit measures at least one selected from the group consisting of pentosidine, carboxyethyl lysine (CEL), and carboxymethyl lysine (CML).

21. The kit for predicting or diagnosing neurodegenerative diseases according to claim 19, wherein the specific protein bound to glycotoxin is collagen.

22. The kit for predicting or diagnosing neurodegenerative diseases according to claim 19, wherein the kit measures a complex of pentosidine, carboxyethyl lysine (CEL) or carboxymethyl lysine (CML) with collagenXVII.

23. A kit for predicting or diagnosing neurodegenerative diseases, measuring the accumulation ratio of the glycotoxin, the specific protein bound to glycotoxin, or the glycotoxin-specific protein complex of claim 1, and methylglyoxal hydroimidazolone (MG-H1).

24. The kit for predicting or diagnosing neurodegenerative diseases according to claim 23, wherein the kit measures at least one selected from the group consisting of CEL/MGH1, CML/MGH1, pentosidine/MGH1, collagen XVII/MGH1, collagen XVII-CEL/ MGH1, collagen XVII-CML/MGH1 and collagen XVII-pentosidine/MGH1.

25. The kit for predicting or diagnosing neurodegenerative diseases according to claim 23, wherein the kit measures [any one of CEL/MGH1, CML/MGH1, pentosidine/MGH1, or collagen XVII/MGH1] and [a complex of collagen XVII with at least one selected from the group consisting of pentosidine, carboxyethyl lysine (CEL), and carboxymethyl lysine (CML)].

26. The kit for predicting or diagnosing neurodegenerative diseases according to claim 23, wherein the kit measures CEL/MGH1 or collagen XVII-CEL.
